# EUROPEAN PATENT APPLICATION

(11) **EP 3 991 722 A1**
(43) Date of publication of application: **04.05.2022**
(21) Application number: 20833235.3
(22) Date of filing: 28.06.2020
(51) Int. Cl.: A61K 9/127, A61K 31/445

(54) **SUSTAINED-RELEASE LIPID COMPOSITION AND PREPARATION METHOD THEREFOR**

(30) Priority: 28.06.2019 CN 201910571807; 30.12.2019 CN 201911390028
(71) Applicant: Jiangsu Hengrui Medicine Co., Ltd., Lianyungang, Jiangsu 222047 (CN); Shanghai Hengrui Pharmaceutical Co., Ltd, Shanghai 200245 (CN)
(72) Inventor: TONG, Xinyong, Shanghai 200245 (CN); ZOU, Aifeng, Shanghai 200245 (CN); DUAN, Ziqing, Shanghai 200245 (CN); WANG, Pingping, Shanghai 200245 (CN); LI, Dong, Shanghai 200245 (CN); HE, Wei, Shanghai 200245 (CN)
(74) Representative: dompatent von Kreisler Selting Werner - Partnerschaft von Patent- und Rechtsanwälten mbB
(86) International application number: PCT/CN2020/098417
(87) International publication number: WO 2020/259670

(57) **Abstract**

Disclosed are a sustained-release lipid composition and a preparation method therefore. Specifically, the present invention relates to a solid composition containing lipids and a liposome composition obtained therefrom, wherein the liposomes have improved release properties.

## Description

The present application claims priority to Chinese patent application No. CN201910571807.3, filed on June 28, 2019, and Chinese patent application No. CN201911390028.X, filed on December 30, 2019, which are incorporated by reference in their entirety for all purposes.

### Technical Field

The present disclosure belongs to the field of pharmaceutics, and relates to a sustained-release lipid composition and a preparation method therefor.

### Background

Liposomes, also known as lipid vesicles, are completely enclosed lipid bilayer membranes that include an internal volume containing an aqueous medium. A lipid bilayer is usually composed of a phospholipid, such as lecithin, and related materials, such as glycolipids. Lipid bilayer membranes generally function in a manner similar to cell membranes. They therefore present some biological properties, such as the ability to be easily accepted into the environment of living cells. Therefore, in recent years, there has been increasing interest in the use of liposomes as carriers for the delivery of compounds with special biological or pharmaceutical properties to patients.

Liposomes can be divided into unilamellar liposomes and multilamellar liposomes by the number of bilayer phospholipid membranes contained in the structure. Vesicles containing unilamellar bilayer phospholipid membranes are called unilamellar vesicles, and vesicles containing multilamellar bilayer phospholipid membranes are called multilamellar vesicles (MLV). The unilamellar vesicles are subdivided into small unilamellar vesicular (SUV) and large unilamellar vesicular (LUV). An MLV is a vesicle in an union-like structure formed by alternated bilayer lipid membranes and water, generally composed of mutilamellar concentric lamellas, and used as a lipid-matrix-based sustained-release drug carrier for local or systemic drug delivery. The methods for preparing MLV generally includes reverse-phase evaporation, thin film hydration, freeze drying, etc.

In recent years, liposomes have been widely applied in drug development due to the property of their sustained-release drug carriers. However, liposomes do not release enough drugs in the initial stage after being delivered into the body, so they cannot produce a good therapeutic effect in the initial stage of administration. At present, there have been some methods to improve the initial release of liposomes. WO2019046293 discloses a method for preparing an MLV for delivering a sustained-release anesthetic composition, in which MLV is prepared by hydrating a highly encapsulated lipid structure (HELS) using a buffer system such as histidine to obtain a partially encapsulated lipid composition. CN1893926B discloses a method for preparing an MLV by mixing an organic phase containing fentanyl and lipids with an aqueous phase, and the final preparation contains 30-40% of fentanyl as a free acid or a free base of a drug, and the remainder (70-60%) in the encapsulated part. CN104666248A discloses a ciprofloxacin preparation for inhalation with dual effects of immediate release mode and sustained release mode, which is prepared by mixing a ciprofloxacin-encapsulated liposome with an unencapsulated ciprofloxacin solution.

### Content of the present invention

The present disclosure aims to provide an improved liposome drug composition to improve the release properties of liposomes.

The present disclosure on one aspect provides a solid composition, including: (1) a lipid, wherein the lipid includes at least one phospholipid; and (2) a free acid or a free base of a drug; and (3) a pharmaceutically acceptable salt, a complex or a chelate of the drug, the pharmaceutically acceptable salt of the drug is preferred.

The free acid or the free base of a drug refers to the active ingredient of the drug existing in the form of noumenon, and it counters to the salt form of the drug.

The pharmaceutically acceptable salt of a drug may be an acidic salt or a basic salt of the drug. The free acid of some drugs can react with an organic acid or an inorganic acid to form an acidic salt. Suitable acidic salts of a drug include, but are not limited to, acetate, benzoate, benzenesulfonate, hydrobromide, camphorsulfonate, hydrochloride, citrate, ethanedisulfonate, fumarate, glucoheptonate, gluconate, glucuronate, hydroiodide, isethionate, lactate, lacturonate, lauryl sulfate, malate, maleate, methanesulfonate, naphthoate, naphthalenesulfonate, nitrate, stearate, oleate, oxalate, pamoate, phosphate, polysemi lacturonate, succinate, sulfate, sulfosalicylate, tartrate, tosylate, trifluoroacetate, etc. of the drug. The free base of some drugs can react with an inorganic base, an organic base, an inorganic salt or an organic salt to form a basic salt. Suitable basic salts of a drug include, but are not limited to, a benzathine salt, a calcium salt, a choline salt, a diethanolamine salt, a diethylamine salt, a magnesium salt, a meglumine salt, a piperazine salt, a potassium salt, a silver salt, a sodium salt, a tromethamine salt, a zinc salt, etc. of the drug.

The solid composition described herein may be powder, granules, or cakes or blocks aggregated together.

In some embodiments, the solid composition is a dry solid composition. The method for drying the composition includes, but is not limited to, evaporation, freeze drying or spray drying.

In some embodiments, the solid composition is subjected to a hydration to form a liposome composition, preferably a liposome composition including multilamellar vesicles (MLV). In some embodiments, SUV or LUV and other liposome forms may also exist in the liposome composition. In some embodiments, the liposome composition exists mainly in the form of MLV.

In some embodiments, the solid composition substantially does not include a lipid vesicle structure, and preferably the solid composition does not include a lipid vesicle structure. In some embodiments, a liposome has not been formed in the solid composition yet.

The operation of the hydration is generally achieved by mixing the solid composition with water or an aqueous solution. In some embodiments, the aqueous solution includes, but is not limited to, an isotonic solution or a buffer. The isotonic solution has an osmotic pressure substantially the same as that of human blood, and it can be prepared from an isotonic agent and water. The isotonic agent includes sodium chloride, potassium chloride, magnesium chloride, calcium chloride, glucose, xylitol and sorbitol. The buffer refers to a buffered solution, which prevents pH value changes through the action of its acid-base conjugated ingredients. In an embodiment, the pH value of the buffer in the present disclosure ranges from about 4.5 to about 8.5. Examples of buffers that can control the pH value in this range include acetate (e.g. sodium acetate), succinate (e.g. sodium succinate), gluconate, histidine, citrate, carbonate, PBS, HEPES or other organic acid buffers.

In some embodiments, the operation of the hydration is achieved by mixing the solid composition with water or an isotonic solution.

In some embodiments, the liposome composition reaches a peak concentration (Cₘₐₓ) of the drug within about 3 hours after being administered to an individual, for example, it reaches the peak concentration (Cₘₐₓ) of the drug within about 2 hours, preferably reaching the peak concentration of the drug within about 1.5 hours, more preferably reaching the peak concentration of the drug within 1 hour, and most preferably reaching the peak concentration of the drug within about 45 minutes.

In some embodiments, the liposome composition formed after hydration provides a sustained release of the drug for not less than 12 hours in an individual, preferably providing the sustained release of the drug for not less than 24 hours, more preferable providing the sustained release of the drug for not less than 48 hours, and most preferably providing the sustained release of the drug for not less than 72 hours.

The lipid described herein may include a completely neutral or negatively charged phospholipid. The term "phospholipid" refers to a hydrophobic molecule containing at least one phosphorus group, which may be natural or synthetic. For example, the phospholipid may contain a phosphorus-containing group and a saturated or unsaturated alkyl group optionally substituted with OH, COOH, oxo, amine or a substituted or unsubstituted aryl group. Phospholipids differ from each other in the length and degree of unsaturation of their acyclic chains.

In some embodiments, the phospholipid includes one or more of phosphatidylcholine, phosphatidylerhanolamine, phosphatidylglycerol, phosphatidylserine, phosphatidic acid, and phosphatidylinositol. The term "phosphatidylcholine" refers to phosphatidylcholine and its derivatives. Examples of the phospholipid suitable for the present disclosure include one or more of dipalmitoylphosphatidylcholine (DPPC), distearoylphosphatidylcholine (DSPC), dimyristoylphosphatidylcholine (DMPC), 1-palmitoyl-2-linoleoyl-sn-glycero-3-phosphatidylcholine (PLPC), dioleoylphosphatidylcholine (DOPC), dioleoylphosphatidylcholine (DEPC), egg yolk phosphatidylcholine (EPC), dilauroylphosphatidylcholine (DLPC), hydrogenated soybean phosphatidylcholine (HSPC), 1-myristoyl-2-palmitoylphosphatidylcholine (MPPC), 1-palmitoyl-2-myristoylphosphatidylcholine (PMPC), 1-palmitoyl-2-stearoylphosphatidylcholine (PSPC), 1-stearoyl-2-palmitoylphosphatidylcholine (SPPC), 1-palmitoyl-2-oleoyl-phosphatidylcholine (POPC), lysophosphatidylcholine, dilinoleoylphosphatidylcholine, distearoylphosphatidylethanolamine (DSPE), dimyristoylphosphatidylethanolamine (DMPE), dipalmitoylphosphatidylethanolamine (DPPE), dioleoylphosphatidylglycerol (DOPG), dimyristoylphosphatidylglycerol (DMPG), distearoylphosphatidylglycerol (DSPG), dipalmitoylglycerol phosphate glycerol (DPPG), dipalmitoylphosphatidylserine (DPPS), 1,2-dioleoyl-sn-glycerol-3-phosphatidylserine (DOPS), dimyristoylphosphatidylserine (DMPS), distearoylphosphatidylserine (DSPS) dipalmitoylphosphatidic acid (DPPA), 1,2-dioleoyl-sn-glycerol-3-phosphatidic acid (DOPA), dimyristoylphosphatidic acid (DMPA), distearoylphosphatidic acid (DSPA), dipalmitoylphosphatidylinositol (DPPI), 1,2-dioleoyl-sn-glycerol-3-phosphatidylinositol (DOPI), dimyristoylphosphatidylinositol (DMPI), and distearoylphosphatidylinositol (DSPI).

In some embodiments, the phospholipid described herein is selected from one or more of dipalmitoylphosphatidylcholine (DPPC), distearoylphosphatidylcholine (DSPC), dioleoylphosphatidylcholine (DOPC), dioleoylphosphatidylcholine (DEPC), dilauroylphosphatidylcholine (DLPC) and dimyristoylphosphatidylcholine (DMPC).

The present disclosure may also include other neutral lipids, cationic lipids and/or anionic lipids.

Examples of other neutral lipids which may be used for the present disclosure include: one or more of steroids such as cholesterol and its derivatives, lecithin, soybean phospholipids, cephalins, sphingomyelin and hydrogenated soybean phospholipids. In some embodiments, relative to a total amount of the lipid mixture, a molar percentage of the steroid was not more than 90%.

In some embodiments, the lipid includes at least one phospholipid and cholesterol. Relative to a total amount of the lipid mixture, a molar percentage of cholesterol may be 0.1%-90%, preferably 10%-80%, and more preferably 20%-70%.

Based on the total moles of the solid composition, the mole content of the lipid may be 10%-99%, preferably 20%-70%.

The "drug" described herein refers to any compound that can be used for preventing, diagnosing, treating or curing diseases, used for relieving pain, or controlling or improving any physiological or pathological disorder of any human or animal. The drug can exist in various forms, such as the free acid or free base form of the drug, the pharmaceutically acceptable salt, complex or chelate form of the drug, etc. The drug described herein include all the forms that can exist. The types of the drug include, but are not limited to: antitumor drugs, antibiotics, antihypertensive drugs, hypoglycemic drugs, antihyperlipidemic drugs, anticonvulsants, antidepressants, antiemetics, antihistamines, anti-tremor paralysis drugs, antipsychotics, anxiolytics, drugs for erectile dysfunction, drugs for migraine, drugs for the treatment of alcoholism, anti-bleeding agents, muscle relaxants, non-steroidal anti-inflammatory agents, anesthetics, analgesics, steroidal anti-inflammatory agents, etc.

Antibiotics include, but are not limited to, cefmetazole, cefazolin, cephalosporin, cefoxitin, cephacetrile, cephalosporin III, cefaloridine, cephalosporin c, cefalothin, cephamycin a, cephamycin b, cephamycin c, cefradine, ampicillin, amoxicillin, hetacillin, carfecillin, carinacillin, carbenicillin, amyl penicillin, azidocillin, benzylpenicillin, clometocillin, cloxacillin, cyclopenicillin, methicillin, nafcillin, 2-pentene penicillin, penicillin n, penicillin o, penicillin s, penicillin v, chlorbutyl penicillin, dicloxacillin, biphenyl, heptyl penicillin, metancillin, etc., and the combinations thereof.

Anticonvulsants include, but are not limited to, 4-amino-3-hydroxybutyric acid, ethanedisulfonate, gabapentin, vigabatrin, etc., and the combinations thereof.

Antidepressants include, but are not limited to, amitriptyline, amoxapine, benzoyl hydrazine, butriptyline, clomipramine, desipramine, dosulepin, doxepin, imipramine, lofepramine, medifoxamine, mianserin, maprotiline, mirtazapine, nortriptyline, protriptyline, trimipramine, viloxazine, citalopram, cotinine, duloxetine, fluoxetine, fluvoxamine, milnacipran, nisoxetine, paroxetine, reboxetine, sertraline, tianeptine, acetaphenazine, binedaline, brofaromine, cericlamine, clovoxamine, isonicotinicacid, isocarboxazid, moclobemide, phenyhydrazine, phenelzine, selegiline, sibutramine, tranylcypromine, ademetionine, adrafinil, amesergide, amisulpride, amperozide, benactyzine, bupropion, caroxazone, gepirone, idazoxan, metralindole, milnacipran, minaprine, nefazodone, nomifensine, ritanserin, roxindole, S-ademetionine, tolfenacin, trazodone, tryptophan, venlafaxine, zalospirone, etc., and the combinations thereof.

Antiemetics include, but are not limited to, alizapride, azasetron, benzquinamide, bromopride, bucrizine, chlorpromazine, cinnarizine, clebopride, ceclizine, diphenhydrazine, difenidol, dolasetron mesylate, haloperidol, granisetron, scopolamine, lorazepam, metoclopramide, metopimazine, ondansetron, perphenazine, promethazine, prochlorperazine, scopolamine, triethylphosphine, trifluoperazine, triflupromazine, trimethobenzamide, tropisetron, domperidone, palonosetron, etc., and the combinations thereof.

Antihistamines include, but are not limited to, azatadine, brompheniramine, clemastine, cyproheptadine, dexmedetomidine, diphenhydramine, doxylamine, hydroxyzine, cetirizine, fexofenadine, loratadine, promethazine, etc., and the combinations thereof.

Anti-tremor paralysis drugs include, but are not limited to, amantadine, baclofen, biperiden, benztropine, orphenadrine, procyclidine, trihexyphenidyl, levodopa, carbidopa, selegiline, deprenyl, apomorphine, benserazide, bromocriptine, budipine, cabergoline, dihydroergocriptine, eliprodil, eptastigmin, ergoline, galantamine, lazabemide, lisuride, mazindol, memantine, mofegiline, pergolide, pramipexole, propentofylline, rasagiline, remacemide, terguride, entacapone, tocapone, etc., and the combinations thereof.

Antipsychotics include, but are not limited to, acephenazine, alizapride, aripiprazole, amperozide, benperidol, benzquinamide, bromoperidol, buramate, butaperazine, carphenazine, carpipramine, chlorpromazine, chlorprothixene, clocapramine, chloramphenicol, clopenthixol, clospirazine, clothiapine, chlormemazine, haloperidol, trifluoroethyl alcohol, fluphenazine, fluspirilene, haloperidol, mesoridazine, methophenazine, penfluridol, piperazine, perphenazine, pimozide, pipamperone, piperacetazine, pipotiazine, prochlorperazine, promazine, remoxipride, sertindole, spiperone, sulpiride, thioridazine, thiothixene, trifluperidol, triflupromazine, trifluoperazine, ziprasidone, zotepine, zuclopenthixol, amisulpride, butaclamol, clozapine, meperone, olanzapine, quetiapine, risperidone, etc., and the combinations thereof.

Anxiolytics include, but are not limited to, mecloqualone, medetomidine, dexmedetomidine, metomidate, adinazolam, chlordiazepoxide, clobenzepam, flurazepam, lorazepam, loprazolam, midazolam, remimazolam, alpidem, alseroxvlon, amphenidone, azacyclonol, bromisoval, buspirone, captodiame, capuride, carchloruria, carbromal, chloral betaine, enciprazine, flesinoxan, ixabepilone, lesopitron, loxapine, methaqualone, propranolol, tandospirone, trazanox, zopiclone, zolpidem, etc., and the combinations thereof.

Drugs for erectile dysfunction include, but are not limited to, tadalafil, sildenafil, vardenafil, apomorphine, apomorphine diacetate, phentolamine, yohimbine, etc., and the combinations thereof.

Anti-bleeding agents include, but are not limited to, thrombin, vitamin K1, protamine sulfate, aminocaproic acid, tranexamic acid, carbazochrome, carbazochrome sodium sulfonate, rutin, hesperidin, etc., and the combinations thereof.

Drugs for migraine include, but are not limited to, almotriptan, codeine, dihydroergotamine, ergotamine, eletriptan, frovatriptan, isometheptene, lidocaine, lisuride, metoclopramide, naratriptan, oxycodone, propoxyphene, rizatriptan, sumatriptan, tolfenamic acid, zolmitriptan, amitriptyline, atenolol, clonidine, cyproheptadine, diltiazem, doxepin, fluoxetine, lisinopril, methysergide, metoprolol, nadolol, nortriptyline, paroxetine, pizotifen, propranolol, protriptyline, sertraline, timolol, verapamil, etc., and the combinations thereof.

Drugs for the treatment of alcoholism include, but are not limited to, acamprosate, naloxone, naltrexone, disulfiram, etc., and the combinations thereof.

Muscle relaxants include, but are not limited to, baclofen, cyclobenzaprine, orphenadrine, quinine, atracurium, rocuronium, succinylcholine, mivacurium bromide, rapacuronium bromide, vecuronium bromide, pancuronium bromide, tizanidine, etc., and the combinations thereof.

Anesthetics include, but are not limited to: ambucaine, amolanone, amylocaine, benoxinate, benzocaine, betoxycaine, xenysalate, bupivacaine, butacaine, butamben, butamben picrate, butanilicaine, butethamine, butoxycaine, carticaine, chloroprocaine, cocaethylene, cocaine, cyclomethycaine, dibucaine, quinisocaine, dimethocaine, diperodon, benzhydrylamine, dyclonine, ecgonidine, ecgonine, ethyl chloride, etidocaine, β-eucaine, hexylcaine, procaine hydrochloride, hydroxyprocaine, hydroxytetracaine, isobucaine, isobutyl p-aminobenzoate, ketocaine, leucine, levoxadrol, mepivacaine, meprylcaine, metabutoxycaine, metabutethamine, myrtecaine, octacaine, orthocaine, oxethazaine, parethoxycaine, phenacaine, Phenol, piperocaine, piridocaine, lauromacrogol 400, pramocaine, prilocaine, procaine, primacaine, proparacaine, propipocaine, propoxycaine, pseudo-cocaine, pyrrocaine, ropivacaine, salicyl alcohol, tetracaine, tolycaine, mesocaine, etc., and the combinations thereof.

Analgesics include, but are not limited to: alfentanil, allylprodine, alphaprodine, anileridine, benzylmorphine, bezitramide, buprenorphine, butorphanol, clonitazene, codeine, cyclazocine, desomorphine, dextromoramide, dextropropoxyphen, dezocine, diampromidum, dihydrocodeine, dihydromorphine, dimenoxadol, dimepheptanol, dimethylthiambutene, dioxaphetyl butyrate, dipipanone, eptazocine, ethoheptazine, ethylmethylthiambutene ethylmorphine, etonitazene, fentanyl, heroin, hydrocodone, hydromorphone, hydroxypethidine, isomethadone, ketobemidone, levallorphan, levorphanol, levophenacylmorphan, lofentanil, pethidine, meptazinol, metazocine, methadone, metopon, morphine, myrophine, nalbuphine, nalorphine, narceinum, nicomorphine, norlevorphanol, normethadone, normorphine, norpipanone, opiates, oxycodone, oxymorphone, papaveretum, pentazocine, phenadoxone, phenazocine, phenomorphan, phenoperidine, piminodine, piritramide, proheptazine, promedol, properidine, propiram, propoxybenzene, sufentanil, tilidine, tramadol, etc., and the combinations thereof.

Steroid anti-inflammatory agents include, but are not limited to: alclometasone, amcinonide, betamethasone, betamethasone 17-valerate, clobetasol, clobetasol propionate, clocortolone, cortisone, dehydrotestosterone, deoxycorticosterone, desonide, desoximetasone, dexamethasone, dexamethasone 21-isonicotinate, diflorasone, fluocinonide, fluocinolone, fluoromethalone, flurandrenolide, fluticasone, halcinonide, ulobetasol, hydrocortisone, hydrocortisone acetate, hydrocortisone cypionate, hydrocortisone hemisuccinate, hydrocortisone 21-lysinate, hydrocortisone succinate, isoflupredone, isoflupredone acetate, methylprednisolone, methylprednisolone acetate, methylprednisolone sodium succinate, methylprednisolone, mometasone, prednicarbate, hydroprednisone, hydroprednisone acetate, hhydroprednisone hemisuccinate, hydroprednisone sodium phosphate, hydroprednisone sodium succinate, hydroprednisone valerate-acetate, prednisone, triamcinolone, triamcinolone acetonide, etc., and the combinations thereof.

Non-steroidal anti-inflammatory agents include, but are not limited to, derivatives of salicylic acid (e.g., salicylic acid, acetylsalicylic acid, methyl salicylate, diflunisal, olsalazine, salsalate, sulfasalazine, etc.), indole and indene acetic acid (e.g., indomethacin, etodolac, sulindac, etc.), fenamic acid (e.g., meclofenamic acid, mefenamic acid, flufenamic acid, niflumic acid, tolfenamic acid, etc.), heteroaryl acetic acid (e.g., acemetacin, alclofenac, clidanac, diclofenac, fenclofenac, fentiazac, furofenac, ibufenac, isoxepac, ketorolac, tiopinac, tolmetin, zidometacin, zomepirac, etc.), derivatives of aryl acetic acid and propionic acid (e.g., alminoprofen, benoxaprofen, bucloxic acid, carprofen, fenbufen, fenoprofen, florfenicol, flurbiprofen, ibuprofen, indoprofen, ketoprofen, miroprofen, naproxen, naproxen sodium, oxaprozin, Pirprofen, pranoprofen, suprofen, tiaprofenic acid, tioxaprofen, etc.), enolic acids (e.g., derivatives of oxicam including ampiroxicam, cinnoxicam, droxicam, lornoxicam, meloxicam, Piroxicam, sudoxicam and tenoxicam; and derivatives of pyrazolone including aminopyrine, antipyrine, apazone, diazepam, oxyphenbutazone, phenylbutazone, etc.), derivatives of p-aminophenol (e.g., acetaminophen, etc.), alkanones (e.g., nabumetone, etc.), nimesulide, proquazone, etc., and the combinations thereof.

In some embodiments, the drug may be an anesthetic, an anti-bleeding agent, an analgesic or a non-steroidal anti-inflammatory agent.

In some embodiments, the drug may be bupivacaine, ropivacaine, butorphanol, dexmedetomidine, tranexamic acid, etc. The free acid or the free base of the drug may be the free base of bupivacaine, the free base of ropivacaine, the free base of butorphanol, the free base of dexmedetomidine, tranexamic acid, etc. The corresponding pharmaceutically acceptable salt, complex or chelate may be the pharmaceutically acceptable salt, complex or chelate of bupivacaine (e.g., bupivacaine hydrochloride), the pharmaceutically acceptable salt, complex or chelate of ropivacaine (e.g., ropivacaine hydrochloride), the pharmaceutically acceptable salt, complex or chelate of butorphanol (e.g., butorphanol tartrate), the pharmaceutically acceptable salt, complex or chelate of dexmedetomidine (e.g., dexmedetomidine hydrochloride), the pharmaceutically acceptable salt, complex or chelate of tranexamic acid, etc.

In some embodiments, the suitable drug described herein includes those with the log P value greater than about 1.0 (namely, the octanol/water distribution coefficient was greater than 10). For example, it may include, as reported, ropivacaine with the log P value of 3.16, bupivacaine with the log P value of 3.69, butorphanol with the log P value of 3.68, fentanyl with the log P value of 4.25, ondansetron with the log P value of 2.37, sumatriptan with the log P value of 1.05, etc.

Based on a total molar amount of the solid composition, a molar content of drug (the free acid or the free base of the drug + the pharmaceutically acceptable salt, the complex or the chelate of the drug) may be 1%-90%, preferably 30%-90%, and more preferably 30%-80%.

The weight percentage of the free acid or the free base of the drug to the pharmaceutically acceptable salt, the complex or the chelate of the drug may be adjusted according to the actually desired therapeutic effect. In some embodiments, a molar ratio of the free acid or the free base of the drug to the pharmaceutically acceptable salt, the complex or the chelate of the drug may be (0.01:1)-(100:1), preferably (1:9)-(9:1).

In some embodiments, the solid composition includes: (1) a lipid including at least one phospholipid; and (2) a ropivacaine free base; and (3) a pharmaceutically acceptable salt of ropivacaine, wherein ropivacaine hydrochloride is preferred.

In some embodiments, the solid composition includes: (1) a lipid including at least one phospholipid; and (2) a bupivacaine free base; and (3) a pharmaceutically acceptable salt of bupivacaine, wherein bupivacaine hydrochloride is preferred.

In some embodiments, the solid composition includes: (1) a lipid including at least one phospholipid; and (2) a butorphanol free base; and (3) a pharmaceutically acceptable salt of butorphanol, wherein butorphanol tartrate is preferred.

In some embodiments, the solid composition includes: (1) a lipid comprising at least one phospholipid; and (2) a dexmedetomidine free base; and (3) a pharmaceutically acceptable salt of dexmedetomidine, wherein dexmedetomidine hydrochloride is preferred.

In some embodiments, the solid composition includes: (1) a lipid including at least one phospholipid; and (2) a free base or a free base of tranexamic acid; and (3) a pharmaceutically acceptable salt of tranexamic acid.

The particle diameter of the phospholipid may be the regular particle diameter of multilamellar vesicles. In some embodiments, the particle diameter d (0.1) of the phospholipid may be greater than 0.5µm, e.g., greater than 1µm. In some embodiments, the particle diameter d (0.5) of the phospholipid may be greater than 1µm, e.g., greater than 5µm, greater than 10µm. In some embodiments, the particle diameter d (0.9) of the phospholipid may be greater than 1µm, e.g., greater than 10µm, greater than 15µm, and greater than 20µm.

In some embodiments, the solid composition includes: (1) a lipid including at least one phospholipid; and (2) a ropivacaine free base; and (3) a pharmaceutically acceptable salt of ropivacaine, wherein ropivacaine hydrochloride is preferred.

In some embodiments, the solid composition is subjected to a hydration to form a liposome composition including multilamellar vesicles (MLV).

In some embodiments, the lipid includes at least one phosphatidylcholine and cholesterol. The phosphatidylcholine is selected from one or more of dipalmitoylphosphatidylcholine (DPPC), distearoylphosphatidylcholine (DSPC), dioleoylphosphatidylcholine (DOPC), dioleoylphosphatidylcholine (DEPC), dilauroylphosphatidylcholine (DLPC) and dimyristoylphosphatidylcholine (DMPC). A molar ratio of the phosphatidylcholine to the cholesterol may be (1000:1)-(1:9), preferably (4:1)-(1:4);

In some embodiments, based on a total molar amount of the solid composition, a molar content of ropivacaine (the free base + the pharmaceutically acceptable salt of ropivacaine) may be 1%-90%, preferably 30%-90%, and more preferably 30%-80%. A molar ratio of the free base to the pharmaceutically acceptable salt of ropivacaine may be (1:0.01)-(0.01:1), preferably (9:1)-(1:9).

In some embodiments, the solid composition includes: (1) a lipid including at least one phosphatidylcholine; (2) a ropivacaine free base; and (3) a pharmaceutically acceptable salt of ropivacaine, and ropivacaine hydrochloride is preferred, wherein,
a molar ratio of the phosphatidylcholine to the cholesterol is (4:1)-(1:4);
a molar ratio of the free base to the pharmaceutically acceptable salt of ropivacaine is (9:1)-(1:9),
preferably, the solid composition substantially does not include a lipid vesicle structure.

In some embodiments, the solid composition is subjected to a hydration to form a liposome composition, preferably a liposome composition including multilamellar vesicles. In some embodiments, the phosphatidylcholine is selected from one or more of dipalmitoylphosphatidylcholine (DPPC), distearoylphosphatidylcholine (DSPC), dioleoylphosphatidylcholine (DOPC), dioleoylphosphatidylcholine (DEPC), dilauroylphosphatidylcholine (DLPC) and dimyristoylphosphatidylcholine (DMPC).

In some embodiments, the solid composition includes: (1) a lipid including at least one phospholipid; and (2) a butorphanol free base; and (3) a pharmaceutically acceptable salt of butorphanol, wherein butorphanol tartrate is preferred.

In some embodiments, the solid composition is subjected to a hydration to form a liposome composition including multilamellar vesicles (MLV).

In some embodiments, the lipid includes at least one phosphatidylcholine and cholesterol. The phosphatidylcholine is selected from one or more of dipalmitoylphosphatidylcholine (DPPC), distearoylphosphatidylcholine (DSPC), dioleoylphosphatidylcholine (DOPC), dioleoylphosphatidylcholine (DEPC), dilauroylphosphatidylcholine (DLPC) and dimyristoylphosphatidylcholine (DMPC), wherein, DMPC was preferred. A molar ratio of the phosphatidylcholine to the cholesterol may be (1000:1)-(1:9), preferably (4:1)-(1:4);

In some embodiments, based on a total molar amount of the solid composition, a molar content of butorphanol (the free base + the pharmaceutically acceptable salt of butorphanol) may be 1%-90%, preferably 30%-90%, and more preferably 30%-80%. A molar ratio of the free base to the pharmaceutically acceptable salt of butorphanol may be (1:0.01)-(0.01:1), preferably (9:1)-(1:9).

The present disclosure also provides a method for preparing the solid composition described herein, including a step of mixing the lipid, the free acid or the free base of the drug, the pharmaceutically acceptable salt, the complex or the chelate of the drug, and a liquid medium; and a step of removing the liquid medium.

The present disclosure also provides a method for preparing the solid composition, including a step of mixing the lipid, the free acid or the free base of the drug, a salt-forming agent, and a liquid medium; and a step of removing the liquid medium.

In some embodiments, the pharmaceutically acceptable salt, the complex or the chelate of the drug may also be added in the step of mixing.

The salt-forming agent includes one or more of the group consisting of an inorganic acid, an organic acid, an inorganic base, an organic base, an inorganic salt, and an organic salt. It may correspond to the pharmaceutically acceptable salt to be formed by the free acid or the free base of the drug.

The suitable salt-forming agents include, but are not limited to, acetic acid, benzoic acid, benzenesulfonic acid, hydrobromic acid, camphorsulfonic acid, acid, citric acid, ethanedisulfonic acid, fumaric acid, glucoheptonic acid, gluconic acid, glucuronic acid, hydroiodic acid, hydroxyethanesulfonic acid, lactic acid, lactobionic acid, lauryl sulfuric acid, malic acid, maleic acid, methanesulfonic acid, naphthoic acid, naphthalenesulfonic acid, nitric acid, stearic acid, oleic acid, oxalic acid, pamoic acid, phosphoric acid, polygalacturonic acid, succinic acid, sulfuric acid, sulfosalicylic acid, tartaric acid, toluenesulfonic acid, trifluoroacetic acid, calcium hydroxide, calcium acetate, calcium chloride, choline, diethanolamine, diethylamine, magnesium hydroxide, magnesium chloride, magnesium methoxide, meglumine, piperazine, potassium hydroxide, sodium hydroxide, tromethamine, zinc chloride, zinc hydroxide, etc.

The suitable liquid medium includes, but is not limited to, water an organic solvent, and a water/organic solvent co-solvent system. The organic solvent includes, but is not limited to, methanol, ethanol, n-propanol, isopropanol, n-butanol, isobutanol, tert-butanol, acetone, acetonitrile, dichloromethane, and dimethyl sulfoxide, etc. The preferable liquid medium can be water, ethanol, isopropanol, tert-butanol, water/ethanol, water/isopropanol, and water/tert-butanol, etc. The liquid medium can be removed by a known technique, such as evaporation, freeze drying or spray drying, preferably freeze drying or spray drying.

In some embodiments, a volume ratio of water to the organic solvent in the water/organic solvent co-solvent may be (1000:1)-(1:1000), preferably (9:1)-(1:1000), and more preferably (1:4)-(1:1000).

The present disclosure also provides a liposome composition obtained by subjecting the solid composition described herein to a hydration.

The operation of the hydration is generally achieved by mixing the solid composition with water or an aqueous solution. In some embodiments, the aqueous solution includes, but is not limited to, an isotonic solution or a buffer. The isotonic solution has an osmotic pressure substantially the same as that of human blood, and it can be prepared from an isotonic agent and water. The isotonic agent includes sodium chloride, potassium chloride, magnesium chloride, calcium chloride, glucose, xylitol and sorbitol. The buffer refers to a buffered solution, which prevents pH changes through the action of its acid-base conjugated ingredients. In an embodiment, the pH of the buffer described herein ranged from about 4.5 to about 8.5. Examples of buffers that can control the pH value in this range include acetate (e.g. sodium acetate), succinate (e.g. sodium succinate), gluconate, histidine, citrate, carbonate, PBS, HEPES or other organic acid buffers.

In some embodiments, the solid composition is subjected to a hydration to form a liposome composition, preferably a liposome composition including multilamellar vesicles (MLV). In some embodiments, SUV or LUV and other liposome forms may also exist in the liposome composition. In some embodiments, the liposome composition exists mainly in the form of MLV.

In some embodiments, the operation of the hydration is achieved by mixing the solid composition with water or an isotonic solution.

In some embodiments, the solid composition described herein or the composition after the hydration may also include other active substances.

In some embodiments, the liposome composition described herein optionally includes a pharmaceutically accepted carrier.

In some embodiments, the liposome composition described herein provides a sustained release of the drug for not less than 12 hours in an individual. In some embodiments, the liposome composition provides a sustained release of the drug for not less than 24 hours. In some embodiments, the liposome composition provides a sustained release of the drug for not less than 36 hours. In some embodiments, the liposome composition provides a sustained release of the drug for not less than 48 hours. In some embodiments, the liposome composition provides a sustained release of the drug for not less than 60 hours. In some embodiments, the liposome composition provides a sustained release of the drug for not less than 72 hours,

In some embodiments, the liposome composition may reach a peak concentration of the drug within about 3 hours after being administered to an individual, for example, it reaches the peak concentration of the drug within about 2 hours, preferably reaching the peak concentration of the drug within about 1.5 hours, more preferably reaching the peak concentration of the drug within 1 hour, and most preferably reaching the peak concentration of the drug within about 45 minutes.

As described herein, the term "individual" includes a mammal and human, and preferably the individual may include a rat. The liposome composition described herein can also produce similar effects. For example, the liposome composition described herein provides a sustained release of the drug for not less than 12 hours in human. In some embodiments, the liposome composition provides a sustained release of the drug for not less than 24 hours. In some embodiments, the liposome composition provides a sustained release of the drug for not less than 36 hours. In some embodiments, the liposome composition provides a sustained release of the drug for not less than 48 hours. In some embodiments, the liposome composition provides a sustained release of the drug for not less than 60 hours. In some embodiments, the liposome composition provides a sustained release of the drug for not less than 72 hours. In some embodiments, the liposome composition provides a sustained release of the drug for not less than 84 hours. In some embodiments, the liposome composition provides a sustained release of the drug for not less than 96 hours. In some embodiments, the liposome composition provides a sustained release of the drug for not less than 108 hours. In some embodiments, the liposome composition provides a sustained release of the drug for not less than 120 hours. In some embodiments, the liposome composition provides a sustained release of the drug for not less than 132 hours. In some embodiments, the liposome composition provides a sustained release of the drug for not less than 144 hours. In some embodiments, the liposome composition provides a sustained release of the drug for not less than 156 hours. In some embodiments, the liposome composition provides a sustained release of the drug for not less than 168 hours. For example, the liposome composition can reach a peak concentration of the drug within about 4 hours after being administered to an individual; in some embodiments, the liposome composition reaches a peak concentration of the drug within about 3.5 hours; in some embodiments, the liposome composition reaches a peak concentration of the drug within about 3 hours; in some embodiments, the liposome composition reaches a peak concentration of the drug within about 2.5 hours; in some embodiments, the liposome composition reaches a peak concentration of the drug within about 2 hours; in some embodiments, the liposome composition reaches a peak concentration of the drug within about 1.5 hours; in some embodiments, the liposome composition reaches a peak concentration of the drug within about 1 hour; in some embodiments, the liposome composition reaches a peak concentration of the drug within about 45 minutes; in some embodiments, the liposome composition reaches a peak concentration of the drug within about 30 minutes; in some embodiments, the liposome composition reaches a peak concentration of the drug within about 15 minutes; in some embodiments, the liposome composition reaches a peak concentration of the drug within about 10 minutes.

In some embodiments, a total concentration of the drug in the liposome composition is about 0.1 mg/mL to about 300 mg/mL, preferably about 1 mg/mL to about 50 mg/mL, and more preferably about 2.5 mg/mL to about 40 mg/mL.

In some embodiments, an unencapsulated drug is about 1% to about 80% of a total molar amount of the drug in the drug composition. In some embodiments, an unencapsulated drug is about 5% to about 70% of a total molar amount of the drug in the drug composition. In some embodiments, an unencapsulated drug is about 10% to about 60% of a total molar amount of the drug in the drug composition. In some embodiments, an unencapsulated drug is about 20% to about 50% of a total molar amount of the drug in the drug composition. In some embodiments, a molar ratio of an unencapsulated drug to an encapsulated drug is (1:50) to (10:1). In some embodiments, a molar ratio of an unencapsulated drug to an encapsulated drug is (1:40) to (5:1). In some embodiments, a molar ratio of an unencapsulated drug to an encapsulated drug is (1:30) to (4:1). In some embodiments, a molar ratio of an unencapsulated drug to an encapsulated drug is (1:20) to (2:1). In some embodiments, a molar ratio of an unencapsulated drug to an encapsulated drug is (1:10) to (1:1). In some embodiments, a molar ratio of an unencapsulated drug to an encapsulated drug is (1: 5) to (1:1).

In some embodiments, the liposome composition is obtained by the solid composition to a hydration; the solid composition includes: (1) a lipid including at least one phospholipid; (2) a ropivacaine free base; and (3) a pharmaceutically acceptable salt of ropivacaine, wherein ropivacaine hydrochloride is preferred.

In some embodiments, the aqueous medium used in the operation of hydration is water or isotonic solution, and the isotonic solution is selected from saline or glucose solution.

In some embodiments, a total concentration of ropivacaine in the liposome composition is about 0.1 mg/mL to about 80 mg/mL, preferably about 1 mg/mL to about 40 mg/mL, and more preferably about 2.5 mg/mL to about 25 mg/mL.

In some embodiments, unencapsulated ropivacaine is about 1% to about 90% of a total molar amount of ropivacaine in the drug composition. In some embodiments, unencapsulated ropivacaine is about 5% to about 50% of a total molar amount of ropivacaine in the drug composition. In some embodiments, unencapsulated ropivacaine is about 10% to about 40% of a total molar amount of ropivacaine in the drug composition. In some embodiments, unencapsulated ropivacaine is about 1% to about 20% of a total molar amount of ropivacaine in the drug composition. In some embodiments, a molar ratio of unencapsulated ropivacaine to encapsulated ropivacaine is (1: 99) to (10:1). In some embodiments, a molar ratio of unencapsulated ropivacaine to encapsulated ropivacaine is (1:40) to (4:1). In some embodiments, a molar ratio of unencapsulated ropivacaine to encapsulated ropivacaine is (1:30) to (4:1). In some embodiments, a molar ratio of unencapsulated ropivacaine to encapsulated ropivacaine is (1:20) to (2:1). In some embodiments, a molar ratio of unencapsulated ropivacaine to encapsulated ropivacaine is (1:10) to (1:1). In some embodiments, a molar ratio of unencapsulated ropivacaine to encapsulated ropivacaine is (1: 5) to (1:1).

In some embodiments, the liposome composition may reach a peak concentration of ropivacaine within about 3 hours after being administered to an individual, for example, it reaches the peak concentration of ropivacaine within about 2 hours, preferably reaching the peak concentration of ropivacaine within about 1.5 hours, more preferably reaching the peak concentration of ropivacaine within 1 hour, and most preferably reaching the peak concentration of ropivacaine within about 45 minutes.

In some embodiments, the liposome composition may provide a sustained release of ropivacaine for not less than 12 hours, preferably providing the sustained release of ropivacaine for not less than 48 hours, more preferable providing the sustained release of ropivacaine for not less than 72 hours, and most preferably providing the sustained release of ropivacaine for not less than 120 hours.

In some embodiments, the solid composition includes: (1) a lipid including at least one phospholipid; and (2) a butorphanol free base; and (3) a pharmaceutically acceptable salt of butorphanol, butorphanol tartrate is preferred.

The liposome composition of drug described herein may be administrated in various ways, including but not limited to local administration, parenteral administration, etc. The composition may include ophthalmic dosage forms and injectable dosage forms, and can include medical diagnostic production.

The present disclosure also provides a method for preparing a liposome composition, including a step of preparing a solid composition, and a step of hydrating the solid composition.

The present disclosure also provides a method for preparing a liposome composition, including a step of preparing a solid composition, and a step of hydrating the solid composition,
wherein, the step of preparing the solid composition includes (1) a step of mixing a lipid, a free acid or a free base of a drug, a pharmaceutically acceptable salt, a complex or a chelate of the drug, and a liquid medium; and a step of removing the liquid medium, or (2) a step of mixing the lipid, the free acid or the free base of the drug, a salt-forming agent, and the liquid medium; and the step of removing the liquid medium.

The lipid, the free acid or the free base of the drug, and the pharmaceutically acceptable salt, the complex or the chelate of the drug are as previously described.

In some embodiments, the pharmaceutically acceptable salt, the complex or the chelate of the drug may also be added in the step of mixing.

The salt-forming agent includes one or more of the group consisting of an inorganic acid, an organic acid, an inorganic base, an organic base, an inorganic salt, and an organic salt. It may correspond to the pharmaceutically acceptable salt to be formed by the free acid or the free base of the drug.

The suitable salt-forming agents include, but are not limited to, acetic acid, benzoic acid, benzenesulfonic acid, hydrobromic acid, camphorsulfonic acid, acid, citric acid, ethanedisulfonic acid, fumaric acid, glucoheptonic acid, gluconic acid, glucuronic acid, hydroiodic acid, hydroxyethanesulfonic acid, lactic acid, lactobionic acid, lauryl sulfuric acid, malic acid, maleic acid, methanesulfonic acid, naphthoic acid, naphthalenesulfonic acid, nitric acid, stearic acid, oleic acid, oxalic acid, pamoic acid, phosphoric acid, polygalacturonic acid, succinic acid, sulfuric acid, sulfosalicylic acid, tartaric acid, toluenesulfonic acid, trifluoroacetic acid, calcium hydroxide, calcium acetate, calcium chloride, choline, diethanolamine, diethylamine, magnesium hydroxide, magnesium chloride, magnesium methoxide, meglumine, piperazine, potassium hydroxide, sodium hydroxide, tromethamine, zinc chloride, zinc hydroxide, etc.

The available liquid medium includes, but is not limited to, water an organic solvent, and a water/organic solvent co-solvent system. The organic solvent includes, but is not limited to, methanol, ethanol, n-propanol, isopropanol, n-butanol, isobutanol, tert-butanol, acetone, acetonitrile, dichloromethane, and dimethyl sulfoxide, etc. The preferable liquid medium can be water, ethanol, isopropanol, tert-butanol, water/ethanol, water/isopropanol, and water/tert-butanol, etc. The liquid medium can be removed by a known technique, such as evaporation, freeze drying or spray drying, preferably freeze drying or spray drying.

In some embodiments, a volume ratio of water to the organic solvent in the water/organic solvent co-solvent may be (1000:1)-(1:1000), preferably (9:1)-(1:1000), and more preferably (1:4)-(1:1000).

The operation of the hydration is generally achieved by mixing the solid composition with water or an aqueous solution. In some embodiments, the aqueous solution includes, but is not limited to, an isotonic solution or a buffer. The isotonic solution has an osmotic pressure substantially the same as that of human blood, and it can be prepared from an isotonic agent and water. The isotonic agent includes sodium chloride, potassium chloride, magnesium chloride, calcium chloride, glucose, xylitol and sorbitol. The buffer refers to a buffered solution, which prevents pH changes through the action of its acid-base conjugated ingredients. In an embodiment, the pH value of the buffer described herein ranges from about 4.5 to about 8.5. Examples of buffers that can control the pH value in this range include acetate (e.g. sodium acetate), succinate (e.g. sodium succinate), gluconate, histidine, citrate, carbonate, PBS, HEPES or other organic acid buffers.

In some embodiments, the operation of the hydration is achieved by mixing the solid composition with water or an isotonic solution.

Some embodiments of the present disclosure relates to methods of treating, ameliorating or preventing diseases, including applying the liposome composition described herein to individuals. In some embodiments, parenteral administration is applied. In some embodiments, local administration is applied. In some embodiments, parenteral administration and local administration are applied. In some embodiments, parenteral administration is selected from intravenous injection, subcutaneous injection, injection into tissues, infiltration at wounds or dripping at wounds.

Standard methods and devices can be used, for example, the liposome composition described herein can be administrated by using a pen, an injection system, a needle and injector, a delivery system for subcutaneous injection port, a tube, etc.

The term of "about" indicates that the quantity, dimension, recipe, parameters and other quantities and properties are not precise and do not need to be precise, but they can be expected approximate values and/or greater values or smaller values, so as to reflect the tolerance, conversion factors, rounded values, measuring errors, and other factors known to those of skill in the art. Its meaning can include changes of ±10%, preferably changes of ±5%.

The solid composition described herein is subjected to a hydration to form a liposome composition, for example, it can form a multilamellar vesicle (MLV). In the prepared liposome composition, a part of the drug is encapsulated in the liposome, and the other part of the drug is unencapsulated. The encapsulated and unencapsulated drugs are in an appropriate ratio, so that after the liposome is administered to an individual, the unencapsulated drug can be released quickly to achieve the therapeutic effect, while the encapsulated drug can provide a sustained release to maintain the therapeutic effect. The ratio of encapsulated to unencapsulated drugs can be determined by measuring the encapsulation rate of the liposome.

The "encapsulation percentage" or "encapsulation rate" of various recipes refers to the percentage of the molar amount of all the drug forms encapsulated in the liposome to the total molar amount of the drug in the composition. Preferably the range of the encapsulation rate is about 1%-99%, more preferably about 60%-85%. The encapsulation rate can be determined by the regular methods for determining encapsulation rates, such as HPLC method.

The term that "it substantially does not include a lipid vesicle structure" refers to that the lipid in the solid composition has not formed a lipid vesicle structure or has not formed a dry lipid vesicle (e.g., the form of lipid vesicles after freeze-drying), for example, no lipid vesicle structure or dry lipid vesicle can be observed in the scanning electron microscope of the solid composition. In some embodiments, the course of forming a lipid vesicle structure is basically not included during the formation of the solid composition. In some embodiments, the lipid vesicle structure contained in the solid composition is not more than about 10%, or not more than 5%, or not more than 3%, or not more than 1%.

The term of "distribution of particle diameter" or "PSD" refers to the distribution of particle diameter in the liposome composition measured by using the dynamic light scattering technology known to those of skill in the art, such as Malvern Mastersizer^{™}2000. The "d (0.1)" described herein refers to the corresponding particle diameter when the accumulated percentage of particle size distribution reaches 10%. The "d (0.5)" described herein refers to the corresponding particle diameter when the accumulated percentage of particle size distribution reaches 50%. The "d (0.9)" described herein refers to the corresponding particle diameter when the accumulated percentage of particle size distribution reaches 90%.

### Brief description of the drawings

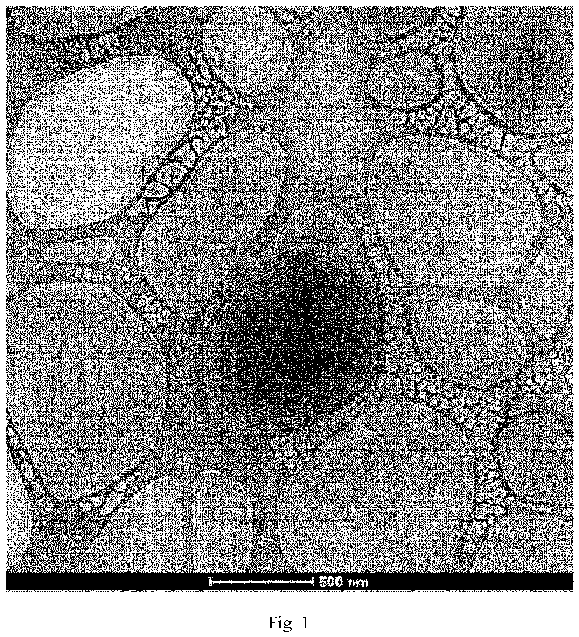

Fig. 1 is the image of the ropivacaine liposome prepared in Embodiment 1 under cryo-transmission electron microscope;
Fig. 2 is the plasma PK curve of the the ropivacaine preparation prepared in Embodiment 1;
Fig. 3 is the in vitro release curve of the ropivacaine liposome prepared in Embodiment 1;
Fig. 4 is the in vitro release curve of the butorphanol liposome prepared in Embodiment 5;
Fig. 5 is the plasma PK curve of the the butorphanol preparation prepared in Embodiment 5;

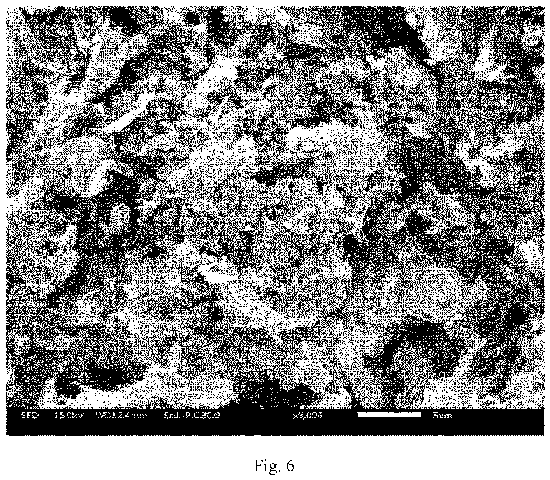

Fig. 6 is the image of the ropivacaine liposome prepared in Embodiment 1 under scanning electron microscope;

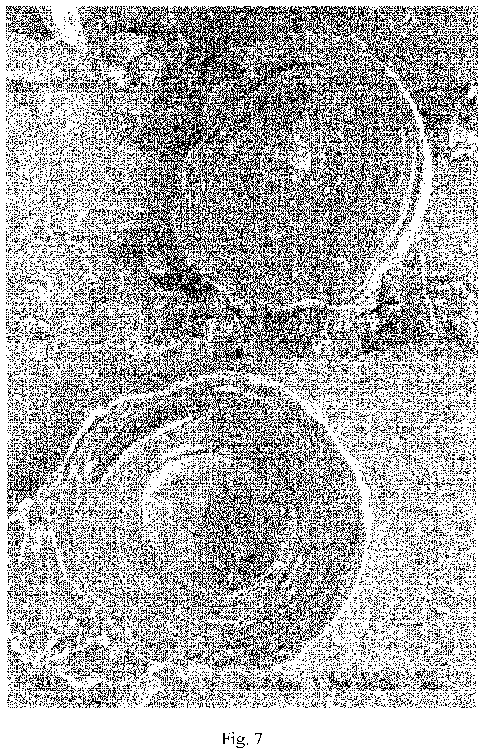

Fig. 7 is the image of the ropivacaine liposome prepared in Embodiment 1 under cryo-scanning electron microscope;
Fig. 8 is the effect of the subcutaneous injection of the ropivacaine liposome prepared in Embodiment 1 on the pain threshold at the administrated site in cavies;
Fig. 9 is the effect of the intracutaneous injection of the ropivacaine liposome prepared in Embodiment 1 on the pain threshold at the administrated site in cavies;
Fig. 10 is the effect of the ropivacaine liposome prepared in Embodiment 1 on the local pain threshold after plantar operation in rats.

### Detailed description of the preferred embodiment

### Embodiment 1: Preparation of a ropivacaine liposome

**Table 1**

| **Name of Material** | **Mass (g)** | **Molar Mass** | **Molar Ratio** |
|---|---|---|---|
| Ropivacaine Free Base | 2.40 | 274.41 | 2.3 |
| Ropivacaine Hydrochloride | 0.68 | 310.88 | 0.6 |
| Dimyristoylphosphatidylcholine (DMPC) | 5.10 | 677.93 | 1.9 |
| Cholesterol | 1.50 | 386.65 | 1.0 |
| Tert-butanol | 100 mL | / | / |

Preparation of a solid composition: 5.10 g of DMPC, 1.50 g of cholesterol, 2.40 g of ropivacaine free base, and 0.68 g of ropivacaine hydrochloride were weighed and dissolved in 100 mL of tert-butanol, and then distributed into 50 penicillin bottles of 10 mL, with 2.0 mL per bottle. The samples were placed in a freeze dryer for freeze drying to prepare the ropivacaine solid composition.

Preparation of a liposome: The freeze dried product was redissolved in the previous stem in an appropriate amount of saline to mix it uniformly. The content and the encapuslation rate of the liposome were determined by HPLC, the content was 17.989 mg/mL (calculated by the ropivacaine free base, the same below), and the encapuslation rate was 79.34%. It could be known from the manifestations under cryo-transmission electron microscope that it included a multilamellar vesicle (MLV). The particle diameter of the liposome was determined to obtain that D [4,3] was 15.959µm, d (0.1) was 3.325µm, d (0.5) was 12.964µm and d (0.9) was 31.674µm.

### Embodiment 2: Pharmacokinetic test of the ropivacaine liposome in rats

### 1. Tested drug

Ropivacaine liposome prepared in Embodiment 1, with a specification of 17.99 mg/ml;

Ropivacaine hydrochloride injection available commercially, with a specification of 10 ml:100 mg (calculated by ropivacaine hydrochloride).

### 2. Experimental animal

Sprague Dawley rats (SPF grade), male, aging about 6-8 weeks, weighing 180-250 g. Original source: Shanghai Sippr-BK Laboratory Animal Co., Ltd. Certification No.: 20180006003423, Production License No.: SCXK (Shanghai) 2018-0006.

### 3. Test steps

The details of grouping and test design are shown in the table below:

**Table 2**

| **Gro up** | **Animal Quantity** | | **Administration** | | | | | |
|---|---|---|---|---|---|---|---|---|
| | **Gen der** | **Quan tity** | **Tested Drug** | **Teste d Drug Dosa ge (mg/ kg)** | **Concentr ation of Tested Drug Solution (mg/mL)** | **Administr ation Volume (mg/kg)** | **Administr ation Manner** | **Collec ted Sampl e** |
| 1 | Male | 5 | Ropivacai ne Hydrochl oride Injection | 19 | 10.0 | 1.9 | Subcutane ous injection | Plasm a |
| 2 | Male | 5 | Ropivacai ne Liposome | 114 | 18.7 | 6.1 | Subcutane ous injection | Plasm a |

Time and volume for sample collection:

Group 1: 6min, 18min, 30min, 45min, 1h, 1.5h, 2h, 3h, 4h, 8h, 12h and 24h after administration. There were a total of 12 time points.

Group 2: Before administration, and 30min, 1h, 2h, 4h, 8h, 24h, 48h, 72h, 96h, 120h, 144h and 168h after administration. There were a total of 12 time points.

A tolerance of 2min was allowed for each time point of blood collection within 30min after administration, a tolerance of 5min was allowed for each time point of blood collection within 30min-8h after administration, a tolerance of 10min was allowed for each time point of blood collection within 8h-48h after administration, and a tolerance of 20min was allowed for each time point of blood collection within 48h-168 after administration.

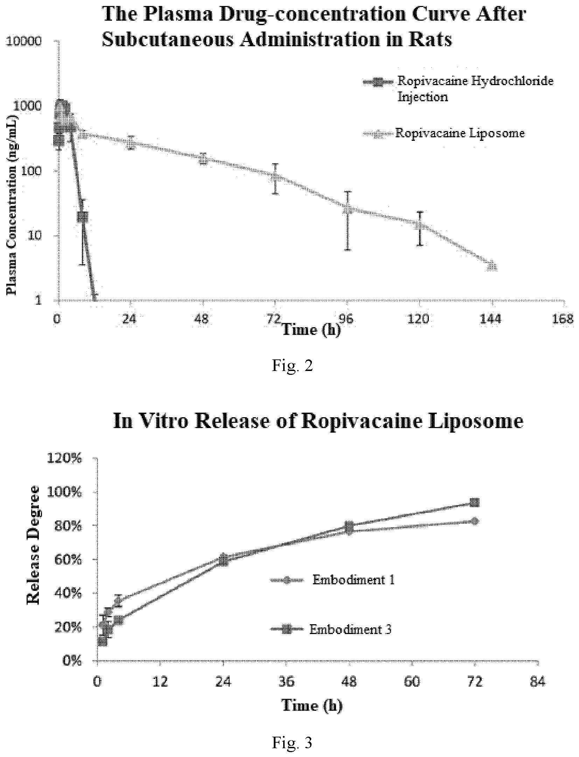

The collected blood samples were placed in heparin anticoagulant blood collection tubes, and centrifuged to separate blood plasma (centrifuge with a centrifugal force of 6800g for 6min, at 2-8°C). The plasma samples were stored in a -80°C refrigerator before sending to the entrusting party. The drug concentration was determined in the plasma by HPLS-MS/MS. The test results are shown in Fig. 2 and the table below.

**Table 3**

| **PK Parameter of Ropivacaine in Plasma of Rats** | | | | | |
|---|---|---|---|---|---|
| | | **Ropivacaine Hydrochloride Injection** | | **Ropivacaine Liposome** | |
| **Dosage** | **mg/kg** | **19** | | **114** | |
| **PK Parameter** | | **Mean** | **SD** | **Mean** | **SD** |
| t_{1/2} | h | 0.851 | 0.08 | 15.1 | 4.84 |
| tₘₐₓ | h | 1.15 | 0.49 | 0.60 | 0.22 |
| Cₘₐₓ | ng·mL⁻¹ | 1078 | 187 | 975 | 259 |
| AUC₀₋ₜ | h·ng·mL⁻¹ | 3967 | 696 | 19825 | 2635 |
| AUC_{0-inf} | h·ng·mL⁻¹ | 3968 | 695 | 19955 | 2663 |

After the rats were given the subcutaneous injection of ropivacaine liposome, the time to peak (tmax) is 0.6h, which is similar to the time to peak (tₘₐₓ=1.15h) of commercially available ropivacaine hydrochloride injections, indicating that the ropivacaine liposome can take effect quickly in vivo, and its onset time in vivo is close to that of the commercially available ropivacaine injections. The half life (t_{1/2}) of ropivacaine liposome is about 18 times of that of the commercially available ropivacaine injections, which proves that the ropivacaine liposome has an ideal sustained-release effect. The administration dosage of the ropivacaine liposome is about 6 times of that of the commercially available preparations, but their peak concentrations (Cₘₐₓ) are similar, which proves that the ropivacaine liposome has a better safety, and its clinically administration dosage can reach at least 6 times of that of the commercially available preparations. In summary, the PK data of the ropivacaine liposome in rats show that this species can not only take effect quickly, but also maintain a longer effect of sustained release, and the higher tolerable dosage is conducive to improving the compliance of patients.

### Embodiment 3: Preparation of a liposome without ropivacaine hydrochloride added initially

**Table 4**

| **Name of Material** | **Mass (g)** | **Molar Mass** | **Molar Ratio** |
|---|---|---|---|
| Ropivacaine Free Base | 3.00 | 274.40 | 2.9 |
| Dimyristoylphosphatidylcholine (DMPC) | 5.10 | 677.93 | 1.9 |
| Cholesterol | 1.50 | 386.65 | 1.0 |
| Tert-butanol | 100 mL | / | / |

According to the method described in Embodiment 1, a liposome was prepared without ropivacaine hydrochloride added initially. The content and the encapuslation rate of the liposome were determined by HPLC; the content was 18.558 mg/mL, and the encapuslation rate was 97.24%.

### Embodiment 4: In vitro release of the ropivacaine liposome

### 1. Cleaning of dialysis bags:

10g of sodium bicarbonate and 0.168g of EDTA•2Na were weighed and added in 500ml of water, and mixed evenly. A dialysis bag was cut into sections of 10cm, and the dialysis bags were washed with 200ml of purified water for 10 times after being boiled with a lotion in a 1L beaker for 10 min; then 500ml of purified water was added to boil for 10 min. The dialysis bags that would be suspended for a long time could be stored in 10% ethanol, and should be washed with 200ml of purified water for 10 times every time before use.

### 2. Preparation of a release medium:

2.3 g of potassium dihydrogen phosphate, 7.6 g of dipotassium hydrogen phosphate trihydrate, 5.6 g of sodium chloride, and 132 mg of ammonium sulfate were weighed and placed in a 1000 ml volumetric flask, water was added to a constant volume, and then it was mixed evenly.

### 3. Placement and sampling determination of released samples:

1.0ml of the liposome solution to be tested was accurately measured using a pipette and added into a 10cm dialysis bag, 1ml of the release medium was added, both ends of the dialysis bag were clamped with clamps, and then it was placed in a 150ml tall beaker containing 99ml of the release medium. The samples were placed on a shaker at 37°C and 10 rpm for incubation, and 1 ml was sampled each time at the set time points for testing by HPLC.

The test results are shown in Fig. 3 and the table below.

**Table 5**

| **Name of Sample** | **In Vitro Release Degree** | | | | | |
|---|---|---|---|---|---|---|
| | **1h** | **2h** | **4h** | **24h** | **48h** | **72h** |
| Embodiment 1 | 21.22% | 28.65% | 35.46% | 61.40% | 76.48% | 82.53% |
| Embodiment 3 | 11.31% | 18.48% | 23.95% | 58.68% | 79.83% | 93.65% |

It can be seen that because of the higher encapsulation rate, the liposome described in Embodiment 3 has a slower release in vitro within 0-4 h, and the release degree at 4h is similar to that of the liposome described in Embodiment 1 at 1h. The above results prove that the higher encapsulation rate will affect the release of the drug at the initial stage, and it is not conducive to the rapid analgesic effect of local analgesics.

### Embodiment 5: Preparation of a butorphanol liposome

**Table 6**

| **Name of Material** | **Mass (g)** | |
|---|---|---|
| | **Prescription 1** | **Prescription 2** |
| Butorphanol Free Base | 3.125 | 3.50 |
| Butorphanol Tartrate | 0.815 | / |
| Dimyristoylphosphatidylcholine (DMPC) | 5.10 | 5.10 |
| Cholesterol | 1.50 | 1.50 |
| Tert-butanol | 100 mL | 100 mL |
| Content | 16.247mg/mL | 16.939mg/mL |
| Encapsulation Rate | 70.35% | 97.76% |

The butorphanol liposome was prepared according to the methods in Embodiment 1 and Embodiment 3.

### Embodiment 6: In vitro release of the butorphanol liposome

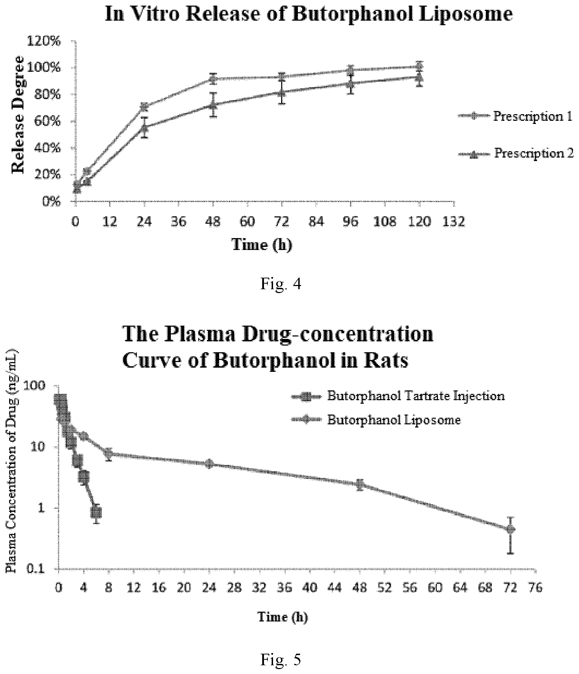

The in vitro release of the butorphanol liposome was determined according to the methods in Embodiment 4. The test results are shown in Fig. 4 and the table below.

**Table 7**

| Name of Sample | In Vitro Release Degree | | | | | | |
|---|---|---|---|---|---|---|---|
| | 0.5h | 4h | 24h | 48h | 72h | 96h | 120h |
| Prescri ption 1 | 12.74% | 22.53% | 70.65% | 91.57% | 93.11% | 98.06% | 100.90% |
| Prescri ption 2 | 9.45% | 14.93% | 55.40% | 72.10% | 81.68% | 88.19% | 93.28% |

The liposome of Prescription 2 has a slower release in vitro within 0-4 h, and its release degree at 4h is similar to that of the liposome described in Prescription 1 at 0.5h. It is was proved that the higher encapsulation rate will affect the release of the drug at the initial stage, and it is not conducive to the rapid analgesic effect of butorphanol.

### Embodiment 7: Pharmacokinetic test of the ropivacaine liposome in rats

### 1. Tested drug

Prescription 1 of the ropivacaine liposome prepared in Embodiment 5, with a specification of 14.96 mg/ml;

Commercially available butorphanol tartrate injection, with a specification of 2 mL: 4 mg.

2. The experimental animals and the test methods are the same as those described in Embodiment 2.

3. Test results: The test results are shown in Fig. 5 and the table below.

**Table 8**

| **PK Parameter of Butorphanol in Plasma of Rats** | | | | | |
|---|---|---|---|---|---|
| | | **Butorphanol Tartrate Injection** | | **Butorphanol Liposome** | |
| **Dosage** | **mg/kg** | **0.5** | | **2** | |
| **PK Parameter** | | **Mean** | **SD** | **Mean** | **SD** |
| t_{1/2} | h | 1.04 | 0.08 | 15.70 | 3.91 |
| tₘₐₓ | h | 0.26 | 0.09 | 0.50 | 0.00 |
| Cₘₐₓ | ng·mL-1 | 64.65 | 3.56 | 28.59 | 3.11 |
| AUC₀₋ₜ | h·ng·mL-1 | 80.40 | 10.20 | 340.39 | 21.72 |
| AUC_{0-inf} | h·ng·mL-1 | 81.68 | 10.55 | 368.63 | 37.45 |

After the rats were given the subcutaneous injection of butorphanol liposome and butorphanol tartrate injection, the times to peak (tₘₐₓ) are all within 0.5h, indicating that the butorphanol liposome can take effect quickly in vivo. The half life (t_{1/2}) of butorphanol liposome is about 15 times of that of the commercially available butorphanol tartrate injection, which proves that the butorphanol liposome has an ideal sustained-release effect. The administration dosage of the butorphanol liposome is about 4 times of that of the commercially available preparations, but peak concentration (Cₘₐₓ) of the butorphanol liposome is lower than the concentration (Cₘₐₓ) of the commercially available butorphanol tartrate injection, which proves that the butorphanol liposome has a better safety, and its clinically administration dosage can reach at least 4 times of that of the commercially available preparations. In summary, the PK data of the butorphanol liposome in rats show that this species can not only take effect quickly, but also maintain a longer effect of sustained release, and the higher tolerable dosage is conducive to improving the compliance of patients.

### Embodiment 8: Analgesic test of the subcutaneous injection of the ropivacaine liposome in cavies

### 1. Tested drug

Ropivacaine liposome prepared in Embodiment 1;

Commercially available ropivacaine hydrochloride injection.

### 2. Test methods

Cavies were divided into the ropivacaine liposome low, middle and high-dosage groups and the ropivacaine hydrochloride injection group, with 10 cavies in each group, including 5 males and 5 females. The cavies in each group were given the subcutaneous injection of the ropivacaine liposome at a dosage of 0.4, 0.885 and 1.9 mg/cavy (approximately equivalent to 1.3, 2.9 and 6.3 mg/kg, calculated by the ropivacaine free base, the same below) and ropivacaine hydrochloride injection at a dosage of 0.885 mg/cavy (approximately equivalent to 2.9 mg/kg) at the upper part of the left rear leg, respectively; the pain threshold at the injected site was determined using the method of acupuncture at 0.5h, 1h, 2h, 4h, 6h, 8h, 10h and 24h after administration.

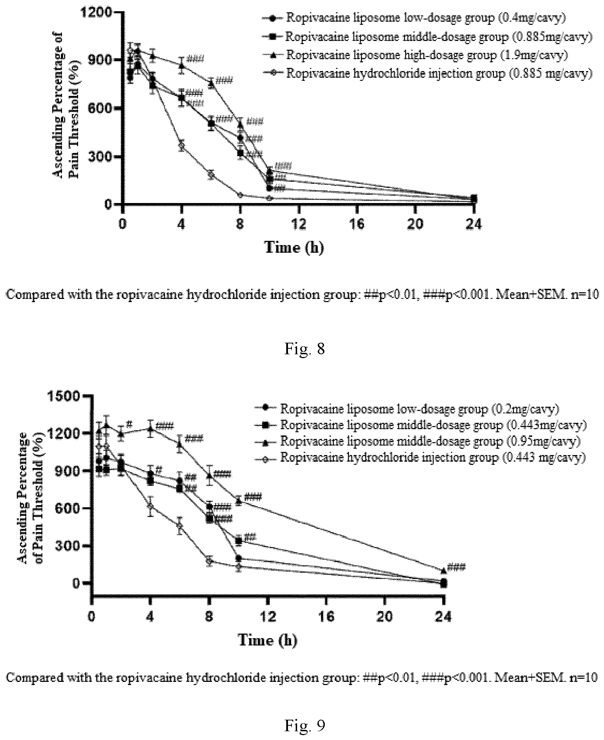

The results are shown in Fig. 8. The pain threshold at the injected site at the first detection point (0.5h) after administration is significantly higher than that before administration (P<0.001); the peak pain threshold is reached at about 1h after administration, and the ascending percentages of the peak pain threshold are 875.70%, 863.35% and 964.28%, respectively; then the pain threshold slowly decreases, but the pain threshold at 24h is still higher than that before administration (P<0.05 or P<0.01), and the drug effect lasts for about 24h. For the cavies given the subcutaneous injection of ropivacaine hydrochloride injection at a dosage of 0.885 mg/cavy, the pain thresholds after administration are significantly higher than that before administration (P<0.001), the peak pain threshold is reached at the first detection point (0.5h) after administration, and the ascending percentage of the peak pain threshold is 961.23%; the pain threshold rapidly decreases from 4h after administration, the pain threshold at 4h after administration is about 45% of the peak pain threshold, the pain threshold at 24h is not obviously different (P>0.05) from that before administration, and the analgesic effect lasts for about 10h. At 4-10h after administration, the pain threshold rapidly decreases in the cavies of the ropivacaine hydrochloride injection group, and the ascending percentage of the peak pain threshold in the cavies of the ropivacaine liposome 0.885 mg/cavy group is significantly higher than that in the ropivacaine hydrochloride injection 0.885 mg/cavy group (P<0.01 or P<0.001).

### Embodiment 9: Analgesic test of the subcutaneous injection of the ropivacaine liposome in cavies

### 1. Tested drug

Ropivacaine liposome prepared in Embodiment 1;

Commercially available ropivacaine hydrochloride injection.

### 2. Test methods

The cavies which completed the administration of subcutaneous injection described in Embodiment 8 were subjected to a 7-day washout period, and then were used for the analgesic test of intracutaneous injection. The animals in each administration group (10 cavies in each group, including 5 males and 5 females) were given the intracutaneous injection of the ropivacaine liposome at a dosage of 0.2, 0.443 and 0.95 mg/cavy (approximately equivalent to 0.7, 1.5 and 3.1 mg/kg, calculated by the ropivacaine free base, the same below) and ropivacaine hydrochloride injection at a dosage of 0.443 mg/cavy (approximately equivalent to 1.5 mg/kg) at the upper part of the left rear leg, respectively; the pain threshold at the injected site was determined using the method of acupuncture at 0.5h, 1h, 2h, 4h, 6h, 8h, 10h and 24h after administration.

The results are shown in Fig. 9. The pain threshold at the injected site at the first detection point (0.5h) after administration is significantly higher than that before administration (P<0.001); the peak pain threshold is reached at about 1h-2h after administration, and the ascending percentages of the peak pain threshold are 1006.41%, 922.63% and 1266.74%, respectively; then the pain threshold slowly decreases, but the pain thresholds in the cavies of the ropivacaine liposome groups at a dosage of 0.2 and 0.443 mg/cavy at 10h after administration are still higher than that before administration (P<0.001), and the pain threshold in the cavies of the ropivacaine liposome group at a dosage of 0.95 mg/cavy at 24h after administration is still higher than that before administration (P<0.001). The analgesic drug effect lasts for about 10h in the cavies given the intracutaneous injection of ropivacaine liposome at a dosage of 0.2 and 0.443 mg/cavy, and the drug effect lasts for about 24h in the cavies given the intracutaneous injection of ropivacaine liposome at a dosage of 0.95 mg/cavy. For the cavies given the intracutaneous injection of ropivacaine hydrochloride injection at a dosage of 0.443 mg/cavy, the pain threshold at the first detection point (0.5h) after administration is significantly higher than that before administration (P<0.001), the peak pain threshold is reached at 1h after administration, and the ascending percentage of the peak pain threshold is 1100.20%; the pain threshold rapidly decreases at 8h after administration, by about 23% of the peak pain threshold; the peak pain threshold at 10h is obviously higher than that before administration, but the pain threshold at 24h is not obviously different from that before administration, and the analgesic effect lasts for about 10h. At 6-10h after administration, the ascending percentages of the peak pain threshold in the cavies of the group given the intracutaneous injection of ropivacaine lipid (0.443 mg/cavy) are significantly higher than those in the ropivacaine hydrochloride injection group (0.443 mg/cavy) (P<0.01 or P<0.001). At 2-24h after administration, the ascending percentages of the peak pain threshold in the cavies of ropivacaine lipid group (0.95 mg/cavy) are significantly higher than those in the ropivacaine hydrochloride injection group (0.443 mg/cavy) (P< 0.05, P<0.01 or P<0.001).

### Embodiment 10: Analgesic test at the plantar operative incision in rats

### 1. Tested drugs

Ropivacaine liposome prepared in Embodiment 1;

Commercially available ropivacaine hydrochloride injection.

### 2. Test methods

Rats were randomly divided by weights into the operation group, the ropivacaine liposome low, middle and high-dosage groups and the ropivacaine hydrochloride injection group, with 10 cavies in each group, including 5 males and 5 females, respectively. The right rear plantar skin was longitudinally incised with a scalpel for the animals of each group, the incisions were about 0.5cm long and about 0.2cm deep, and then the incisions were sutured. On Day 2 postoperatively, the animals in each group were given the plantar injection of ropivacaine liposome blank preparation, ropivacaine liposome (0.2, 0.443 and 0.95 mg/rat, approximately equivalent to 0.9, 1.9 and 4.2 mg/kg) and ropivacaine hydrochloride injection at a dosage of 0.443 mg/rat (approximately equivalent to 1.9 mg/kg), respectively; the pain threshold at the injected site was determined using the method of acupuncture at 0.5h, 1h, 2h, 4h, 6h, 8h, 10h and 24h after administration.

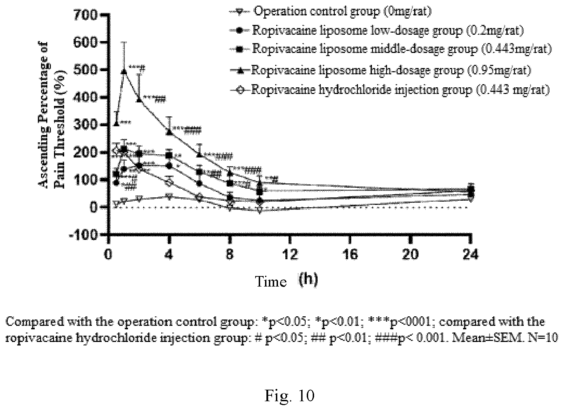

The results are shown in Fig. 10. After the plantar operation, the plantar pain threshold in the rats decreases obviously, and the descending percentages of the pain threshold are about 60%. At 0.5-10h after the plantar injection, the pain thresholds in the rats of the ropivacaine liposome low, middle and high-dosage groups (0.2, 0.443 and 0.95 mg/rat) are significantly higher than that in the operation control group (P<0.05, P<0.01 or P<0.001), representing a dosage-independent at each time point; the analgesic effect of ropivacaine liposome lasts for about 10h. For the rats given the plantar injection of ropivacaine hydrochloride injection at a dosage of 0.443 mg/rat, the pain thresholds at 0.5h-2h after administration are significantly higher than those in the operative control group (P<0.01 or P<0.001), and the analgesic drug effect lasts for about 2h. For the rats given the plantar injection of ropivacaine liposome at a dosage of 0.443 mg/rat, the ascending percentages of the pain threshold at 6h-8h after administration are significantly higher than those in the ropivacaine hydrochloride injection group ( 0.443 mg/rat) (P<0.05 or P<0.01). For the rats given the plantar injection of ropivacaine liposome at a dosage of 0.95 mg/rat, the ascending percentages of the peak pain threshold at 1h-10h are significantly higher than those in the ropivacaine hydrochloride injection group (0.443 mg/rat) (P< 0.05, P<0.01 or P<0.001).

### Embodiment 11: A ropivacaine liposome

**Table 9**

| **Name of Material** | **Mass** | | |
|---|---|---|---|
| | L2 | L3 | L4 |
| Ropivacaine Free Base | 3.23g | 3.23g | 3.23g |
| Ropivacaine Hydrochloride | 0.65g | 0.65g | 0.65g |
| DMPC | 1.62g | | |
| DEPC | | 8.58g | |
| DLPC | | | 5.94g |
| Cholesterol | 0.92g | 1.85g | 1.85g |
| Tert-butanol | 80ml | 80ml | 80ml |
| Water | 20ml | 20ml | 20ml |
| Content of Active Substance | 18.335mg/ml | 19.095mg/ml | 19.798mg/ml |
| Encapsulation Rate | 83% | 83% | 84% |

Phospholipid, cholesterol, ropivacaine free base, and ropivacaine hydrochloride were weighed and dissolved in 100ml of tert-butanol-water mixed solvent, and then distributed into 20ml vials, 5ml per bottle. The samples were placed in a freeze dryer for freeze drying. The freeze dried product was redissolved in an appropriate amount of saline, and mixed uniformly to obtain the target liposome. The content of active substance and the encapsulation rate were determined.

### Embodiment 12

The in vitro release of the ropivacaine liposome L2-L4 was determined using the test methods described in Embodiment 4. The test results are shown in the table below.

**Table 10**

| Name of Sample | In Vitro Release Degree | | | | | |
|---|---|---|---|---|---|---|
| | 2h | 4h | 24h | 48h | 72h | 96h |
| L2 | 24.00% | 29.55% | 58.17% | 74.77% | 84.69% | 90.42% |
| L3 | / | 22.92% | 56.72% | 75.32% | 83.50% | 91.84% |
| L4 | 18.91% | 25.66% | 50.28% | 66.95% | 77.65% | 84.77% |

Although the preferred embodiments of the present disclosure are described above, those of skill in the art should be understood that, these are illustrations only, and under the premise of not deviating from the principle and essence of the present invention, various changes or modifications can be achieved to these embodiments. Therefore, the protective scope of the present invention is defined by the claims attached.

## Claims

1. A solid composition, comprising: (1) a lipid, wherein the lipid comprises at least one phospholipid; and (2) a free acid or a free base of a drug; and (3) a pharmaceutically acceptable salt, a complex or a chelate of the drug, wherein the pharmaceutically acceptable salt of the drug is preferred.

2. The solid composition according to claim 1, wherein the phospholipid comprises one or more of phosphatidylcholine, phosphatidylerhanolamine, phosphatidylglycerol, phosphatidylserine, phosphatidic acid, and phosphatidylinositol, preferably one or more of dipalmitoylphosphatidylcholine, distearoylphosphatidylcholine, dimyristoylphosphatidylcholine, 1-palmitoyl-2-linoleoyl-sn-glycero-3-phosphatidylcholine, dioleoylphosphatidylcholine, egg yolk phosphatidylcholine, dielaidinoyl phosphatidylcholine, dilauroylphosphatidylcholine, hydrogenated soybean phosphatidylcholine, 1-myristoyl-2-palmitoylphosphatidylcholine, 1-palmitoyl-2-myristoylphosphatidylcholine, 1-palmitoyl-2-stearoylphosphatidylcholine, 1-stearoyl-2-palmitoylphosphatidylcholine, 1-palmitoyl-2-oleoyl-phosphatidylcholine, lysophosphatidylcholine, dilinoleoylphosphatidylcholine, distearoylphosphatidylethanolamine, dimyristoylphosphatidylethanolamine, dipalmitoylphosphatidylethanolamine, dioleoylphosphatidylglycerol, dimyristoylphosphatidylglycerol, distearoylphosphatidylglycerol, dipalmitoylglycerol phosphate glycerol, dipalmitoylphosphatidylserine, 1,2-dioleoyl-sn-glycerol-3-phosphatidylserine, dimyristoylphosphatidylserine, distearoylphosphatidylserine, dipalmitoylphosphatidic acid, 1,2-dioleoyl-sn-glycerol-3-phosphatidic acid, dimyristoylphosphatidic acid, distearoylphosphatidic acid, dipalmitoylphosphatidylinositol, 1,2-dioleoyl-sn-glycerol-3-phosphatidylinositol, dimyristoylphosphatidylinositol, and distearoylphosphatidylinositol, and more preferably one or more of dipalmitoylphosphatidylcholine, distearoylphosphatidylcholine, dielaidinoyl phosphatidylcholine, dioleoylphosphatidylcholine, dilauroylphosphatidylcholine, and dimyristoylphosphatidylcholine.

3. The solid composition according to claim 1, wherein the lipid further comprises a steroid, preferably cholesterol.

4. The solid composition according to claim 3, wherein relative to a total molar amount of the lipid, a molar percentage of the cholesterol is 0.1%-90%, preferably 10%-80%, and more preferably 20%-70%.

5. The solid composition according to claim 1, wherein based on a total molar amount of the solid composition, a total content of the free acid or the free base of the drug and the pharmaceutically acceptable salt, the complex or the chelate of the drug is 1%-90%, preferably 30%-90%, and more preferably 30%-80%.

6. The solid composition according to claim 1, wherein a molar ratio of the free acid or the free base of the drug to the pharmaceutically acceptable salt, the complex or the chelate of the drug is (0.01:1)-(100:1), preferably (1:9)-(9:1).

7. The solid composition according to claim 1, wherein the drug is selected from the group consisting of an anesthetic, an anti-bleeding agent, an analgesic, and a non-steroidal anti-inflammatory agent.

8. The solid composition according to any one of claims 1-7, wherein the solid composition comprises: (1) a lipid comprising at least one phospholipid; and (2) a ropivacaine free base; and (3) a pharmaceutically acceptable salt of ropivacaine, wherein ropivacaine hydrochloride is preferred.

9. A solid composition, comprising: (1) a lipid, wherein the lipid comprises at least one phosphatidylcholine and cholesterol; and (2) a ropivacaine free base; and (3) a pharmaceutically acceptable salt of ropivacaine, wherein ropivacaine hydrochloride is preferred; wherein
a molar ratio of the phosphatidylcholine to the cholesterol is (4:1)-(1:4);
a molar ratio of the free base to the pharmaceutically acceptable salt of ropivacaine is (9:1)-(1:9),
preferably, the solid composition substantially does not comprise a lipid vesicle structure.

10. The solid composition according to any one of claims 1-9, wherein the solid composition substantially does not comprise a lipid vesicle structure.

11. The solid composition according to any one of claims 1-10, wherein the solid composition is subjected to a hydration to form a liposome composition, preferably a liposome composition comprising multilamellar liposomes.

12. The solid composition according to claim 11, wherein an operation of the hydration is achieved by mixing the solid composition with water or an aqueous solution, preferably, the aqueous solution is selected from an isotonic solution or a buffer.

13. The solid composition according to claim 11, wherein the liposome composition reaches a peak concentration of the drug within about 3 hours after being administered to an individual, preferably reaching the peak concentration of the drug within about 1.5 hours, more preferably reaching the peak concentration of the drug within 1 hour, and most preferably reaching the peak concentration of the drug within about 45 minutes, preferably, the individual is selected from an mammal and human.

14. The solid composition according to claim 11, wherein the liposome composition provides a sustained release of the drug for not less than 12 hours in an individual, preferably providing the sustained release of the drug for not less than 24 hours, more preferable providing the sustained release of the drug for not less than 48 hours, and most preferably providing the sustained release of the drug for not less than 72 hours, preferably, the individual is selected from an mammal and human.

15. A method for preparing the solid composition according to any one of claims 1-14, comprising a step of mixing the lipid, the free acid or the free base of the drug, the pharmaceutically acceptable salt, the complex or the chelate of the drug, and a liquid medium; and a step of removing the liquid medium.

16. A method for preparing the solid composition according to any one of claims 1-14, comprising a step of mixing the lipid, the free acid or the free base of the drug, a salt-forming agent, and a liquid medium; and a step of removing the liquid medium.

17. The method according to claim 16, wherein the salt-forming agent is selected from one or more of the group consisting of an inorganic acid, an organic acid, an inorganic base, an organic base, an inorganic salt, and an organic salt.

18. The method according to any one of claims 15-17, wherein the liquid medium is selected from the group consisting of water, an organic solvent, and a water/organic solvent co-solvent system, preferably one or more of water, methanol, ethanol, n-propanol, isopropanol, n-butanol, isobutanol, tert-butanol, acetone, acetonitrile, dichloromethane, and dimethyl sulfoxide, more preferably water, ethanol, isopropanol, tert-butanol, water/ethanol, water/isopropanol, and water/tert-butanol, and a volume ratio of water to the organic solvent in the water/organic solvent co-solvent is preferably (1000:1)-(1:1000), more preferably (9:1)-(1:1000), and most preferably (1:4)-(1:1000).

19. The method according to any one of claims 15-17, wherein a method for removing the liquid medium is evaporation, freeze drying or spray drying, preferably freeze drying or spray drying.

20. A liposome composition obtained by the solid composition according to any one of claims 1-14 to a hydration.

21. The liposome composition according to claim 20, wherein an operation of the hydration comprises mixing the solid composition with water or an aqueous solution, and the aqueous solution is preferably an isotonic solution or a buffer.

22. The liposome composition according to claim 20, wherein a total concentration of the drug in the liposome composition is about 0.1 mg/mL to about 300 mg/mL, preferably about 1 mg/mL to about 50 mg/mL, and more preferably about 2.5 mg/mL to about 40 mg/mL.

23. The liposome composition of claim 20, wherein an unencapsulated drug is about 1% to about 90% of a total molar amount of the drug in the liposome composition.

24. The liposome composition according to claim 20, wherein a molar ratio of an unencapsulated drug to an encapsulated drug is (1:99) to (10:1).

25. The liposome composition according to claim 20, wherein the liposome composition reaches a peak concentration of the drug within about 3 hours after being administered to an individual, preferably reaching the peak concentration of the drug within about 1.5 hours, more preferably reaching the peak concentration of the drug within 1 hour, and most preferably reaching the peak concentration of the drug within about 45 minutes, preferably, the individual is selected from an mammal and human.

26. The liposome composition according to claim 20, wherein the liposome composition provides a sustained release of the drug for not less than 12 hours in an individual, preferably providing the sustained release of the drug for not less than 24 hours, more preferable providing the sustained release of the drug for not less than 48 hours, and most preferably providing the sustained release of the drug for not less than 72 hours, preferably, the individual is selected from an mammal and human.

27. A method for preparing a liposome composition, comprising the steps of preparing the solid composition according to any one of claims 15-19, and a step of hydrating the solid composition.

28. A method for preparing a liposome composition, comprising a step of preparing a solid composition, and a step of hydrating the solid composition, wherein:
the step of preparing the solid composition comprises (1) a step of mixing a lipid, a free acid or a free base of a drug, a pharmaceutically acceptable salt, a complex or a chelate of the drug, and a liquid medium; and a step of removing the liquid medium, or (2) a step of mixing the lipid, the free acid or the free base of the drug, a salt-forming agent, and the liquid medium; and the step of removing the liquid medium.
